(19) Europäisches Patentamt
European Patent Office
Office européen des brevets

(11) EP 2 892 621 B1

(12) EUROPEAN PATENT SPECIFICATION

(45) Date of publication and mention of the grant of the patent:
**21.12.2016 Bulletin 2016/51**

(21) Application number: **13759216.8**

(22) Date of filing: **09.09.2013**

(51) Int Cl.:
***A61Q 19/10*** (2006.01) ***A61K 8/02*** (2006.01)
***A61K 8/81*** (2006.01) ***A61K 8/73*** (2006.01)
***A61K 8/25*** (2006.01) ***A61K 8/04*** (2006.01)

(86) International application number:
**PCT/EP2013/068562**

(87) International publication number:
**WO 2014/037553 (13.03.2014 Gazette 2014/11)**

# (54) SURFACTANT AND SOLVENT-FREE HEAVY DUTY SKIN CLEANSER

TENSID UND LÖSUNGSMITTELFREIER INTENSIVHAUTREINIGER

NETTOYANT PUISSANT POUR LA PEAU SANS TENSIOACTIF ET SANS SOLVANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.09.2012 US 201261697983 P**

(43) Date of publication of application:
**15.07.2015 Bulletin 2015/29**

(73) Proprietor: **Deb IP Limited**
**Denby, Derbyshire DE5 8JZ (GB)**

(72) Inventor: **GRASCHA, Pierre Bruno**
**F-51350 Cormontreuil (FR)**

(74) Representative: **Elsy, David**
**Withers & Rogers LLP**
**4 More London Riverside**
**London SE1 2AU (GB)**

(56) References cited:
**EP-A1- 0 635 260**
**EP-A1- 1 627 624**
**WO-A2-2012/084780**
**JP-A- 2002 322 043**
**US-B2- 6 770 285**

- **TODD OSTERGAARD ET AL: "Next-Generation Rheology Control Brings New Formulating Options for Personal Care", 20120101, 1 January 2012 (2012-01-01), pages 1-6, XP007921836,**
- **DATABASE GNPD [Online] MINTEL; February 2003 (2003-02), Anonymous: "Body Wash", XP002727132, Database accession no. 189857**
- **M Karsheva ET AL: "THE CHOICE OF THE THICKENER -A WAY TO IMPROVE THE COSMETICS SENSORY PROPERTIES", , 1 January 2007 (2007-01-01), pages 187-194, XP055128800, Retrieved from the Internet: URL:http://www.uctm.edu/journal/j2007-2/10 -Kursheva_187-194.pdf [retrieved on 2014-07-14]**
- **MARIOS HOPKINS HATZOPOULOS ET AL: "Are Hydrotropes Distinct from Surfactants?", LANGMUIR, vol. 27, no. 20, 18 October 2011 (2011-10-18), pages 12346-12353, XP055128292, ISSN: 0743-7463, DOI: 10.1021/la2025846**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 892 621 B1

## Description

The present invention relates to the field of skin and surface cleansing formulations, more specifically to surfactant-free and solvent-free heavy duty skin cleansers.

## BACKGROUND

Heavy duty skin cleansers are used for removing difficult to eliminate hydrophobic stains such as waterproof make-up, oil, grease, and tar to mention just a few. These formulations combine surfactants (to emulsify) and/or solvents (to solubilize), and sometimes fine abrasives particles (for example synthetic microbeads, ground fruit stones, minerals, vegetable fibers) with mechanical properties. The combination of the three modes of action - emulsification, solubilisation and scrubbing - makes these products particularly effective.
A typical composition of heavy duty skin cleanser consists of 5 to 20% of surfactants (anionics, and/or amphoterics and/or non-ionics), 1 to 30% of a solvent (for example white spirits, vegetable oils, esters, paraffin oil, glycols and terpenes) and 1 to 10% of microbeads (from vegetable, synthetic or mineral origins).
Surfactants and non-polar or semi-polar solvents have various levels of human and environmental toxicity. For instance, some surfactants may be irritating to the skin (i.e. sodium lauryl sulfate, and alkaline soaps) and solvents may be neurotoxic (i.e. hydrocarbons), hematotoxic (i.e. glycol ethers), allergising (i.e. oxidized terpenes, lactone sesquiterpenes), mutagenic (i.e. aromatics).
Therefore, one way to improve the toxicological and environmental profile of skin cleansers would be to reduce significantly their total amount of active matter (surfactants and solvents), assuming that highly synergistic associations of ingredients may be formulated; this is easier said than done, simply because thousands of formulators have already been working on such projects for the last 50 years, all over the world.
Thus it would be very beneficial to develop a totally new concept of hydrophobic stain removers by using a safe substitute to surfactants and solvents.

## SUMMARY

Provided herein are skin cleansing formulations particularly useful for cleaning heavy duty oil/dirt from skin based on lipophilic particles as a hydrophobic stain-removing agent for the replacement of all or part of the surfactants and solvents normally involved in greasy stain-removing heavy duty skin cleansers.
The present invention is defined by the independent claims. Specific embodiments are defined in the dependent claims.
An embodiment of a skin cleansing formulation is a gel in which lipophilic polymeric particles are suspended. The particles are present in a concentration from about 0.1 % w/w to about 99% w/w of the formulation, and the gel comprises at least one non-Newtonian thickener present in an amount sufficient to provide the formulation with a yield value of at least 30 dynes/$cm^2$.
In embodiments, the formulation is free of lipophilic solvents and/or is free of surfactants.
A Newtonian thickener can be any one or more of acrylates/CI O-30 alkyl acrylate crosspolymer, carbomer, xanthan gum, guar gum, quaternised guar gum, alginate, bentonite, and is typically present in a concentration totalling from about 0.01 to about 10% w/w of the formulation.
The polymer particles are often present in the formulation in a range from about 0.5 to about 20% w/w of the formulation, for example, or range from about 1 to about 10% w/w of the formulation.
While lipophilic particles may themselves act as scrubbing particles, a formulation may additionally include other scrubbing particles in a concentration from about 0.1 to about 95% w/w, more likely in a concentration from about 0.5 to about 20% w/w, or from about 1 to about 10% w/w of the formulation. Such scrubbing particles can be any one or any combination of vegetable based, organic based and mineral based scrubbing agents, such as, for example, vegetable based particles e.g., one or more of ground cornmeal, ground fruit stones, ground walnuts, or other organic based scrubbing particles such as wood dust, polyethylene and/or polypropylene, or mineral based scrubbing particles such as pumice and/or ground shellfish.
One or more preservatives having bacteriostatic and/or fongistatic properties can be included in a formulation.
A formulation can also include one or more than one chelating agent, typically in a concentration of from 0.01 to 5% w/w, or more likely from about 0.1 to about 0.5% w/w of the formulation.
A formulation can include skin conditioner(s) such as polyols (glycerin, polyglycerol, sorbitol, and propylene glycol), allantoin, quaternised polymers or gums (polyquaterniums, dihydroxypropyl PEG-5 linoleammonium chloride, guar hydroxypropyltrimonium chloride, behentrimonium chloride), butylene glycol, hydrogenated vegetal oils, and tocopherols. Typical conditioner(s) concentrations are from about 0.01 to about 10% w/w or from about 0.1 to about 5% w/w of the formulation.

As discussed further below, a formulation can include one or more hydrotrope in a concentration of from about 0.01 to about 10% w/w, or from about 0.1 to about 5% w/w of the formulation.

Lipophilic particles of a formulation generally have a size in a range from about 10 to about 3000 μm, or from about 50 μm to about 1000 μm. An average size of the particles can be in the range of from 50 μm to 2000 μm, or they can be microparticles having an average size in the range of from 50 μm to 900 μm, 100 μm to 700 μm, or from 200 μm to 600 μm.

A formulation can thus be substantially free of lipophilic solvent and/or substantially free of surfactant.

Formulations to which no water has been added are also possible.

Where water is a component of a gel formulation, it may be present in amount of from about 0% to about 85% w/w or more of the formulation.

A pH adjuster may also be present, to bring the pH of the formulation into a range of e.g., between about 4 and about 9, more typically from about 5 to about 7.

Lipophilic particles include one or more polymers of a norbornane, norbornene, norbornadiene, or derivatives of any of these, cellulose acetate; aggregates of fumed silica, or a combination of any of the foregoing.

A derivative of norbornane, norbornene or norbornadiene include those in which a hydrogen atom of one more C-H bonds of norbornane, norbornene or norbornadiene is substituted with a hydrocarbyl group, as discussed further below.

An example described in greater detail below involves a formulation containing poly(norbornene), a homopolymer of of bicyclo[2.2.1]hept-2-ene (norbornene).

Another embodiment of a skin cleansing formulation comprises a gel and particles of a polymer comprised of any one of norbornadiene, its derivatives, norbornane and its derivatives, with the particles being suspended in the gel and present in a concentration from about 0.1 % to about 99% w/w of the formulation.

Another embodiment of the invention is a skin cleansing formulation comprising lipophilic polymeric particles wherein the formulation is substantially free of lipophilic solvent, substantially free of surfactant, and particles form from 0.1% to about 100% of the formulation.

Water can be included in such a formulation in an amount of about 1% to about 95.5% w/w, about 10% to about 95%, about 15% to about 95%, about 20% to about 95%, about 30% to about 95%, about 40% to about 95%, about 50% to about 95%, about 1% to about 90%, about 1% to about 80%, about 1% to about 70%, about 1% to about 60%, about 5% to about 95%, about 5% to about 80%, about 20% to about 95%, about 20% to about 80%, about 30% to about 95%, about 30% to about 80%, about 40% to about 99%, about 40% to about 95%, about 40% to about 80%, or about 50% to about 95%, w/w of the formulation.

The formulation can include scrubbing particles, as described above, e.g., in a concentration from about 0.5 to about 20% w/w of the formulation.

The formulation can include preservative(s) as described in connection with gel formulations.

The formulation can include chelating agent(s), as described above.

The formulation can include one or more skin conditioners as described elsewhere herein.

The formulation can also include at least one hydrotrope as described below.

Lipophilic particles of the formulation can have a size in a range from about 10 to about 3000 μm and can be microparticles having a size in a range from about 50 μm to about 1000 μm, or as elsewhere described herein.

A formulation containing water may include a pH-adjuster.

The lipophilic particles can comprise any of those described above or as set out in the detailed description, including a polymer of a norbornane, norbornene, norbornadiene, or a derivative of norbornane, norbornene or norbornadiene; cellulose acetate; aggregates of fumed silica, or a combination of any of these.

In aspects, the polymer particles are present in the formulation in a range from about 0.5 to about 20% w/w or from about 1 to about 10% w/w of the formulation.

The invention includes a composition comprising a formulation as described herein for use as a hand cleanser, particularly a heavy duty cleanser, including a method of cleansing hands using such a composition.

A further understanding of the functional and advantageous aspects of the disclosure can be realized by reference to the following detailed description and drawings.

## BRIEF DESCRIPTION OF THE TABLES

**Table 1** shows a formulation used to test effectiveness of heavy duty hand cleansers;
**Table 2** shows an exemplary test formulation;
**Table 3** summarizes the final scores used to rate different formulations;
**Table 4** shows non-limiting examples of water-based formulations that can be used as formulations for a gritty foam

dispenser in accordance with the present invention; and

**Tables 5 to 10** show non-limiting examples of water-less formulations.

## DETAILED DESCRIPTION

Various embodiments and aspects of the disclosure will be described with reference to details discussed below. The following description and drawings are illustrative of the disclosure and are not to be construed as limiting the disclosure. The drawings are not necessarily to scale. Numerous specific details are described to provide a thorough understanding of various embodiments of the present disclosure. However, in certain instances, well-known or conventional details are not described in order to provide a concise discussion of embodiments of the present disclosure.

As used herein, the terms, "comprises" and "comprising" are to be construed as being inclusive and open ended, and not exclusive. Specifically, when used in this specification including claims, the terms, "comprises" and "comprising" and variations thereof mean the specified features, steps or components are included. These terms are not to be interpreted to exclude the presence of other features, steps or components.

As used herein, the terms, **"comprises"** and **"comprising"** are to be construed as being inclusive and open ended, and not exclusive. Specifically, when used in this specification including claims, the terms, "comprises" and "comprising" and variations thereof mean the specified features, steps or components are included. These terms are not to be interpreted to exclude the presence of other features, steps or components.

As used herein, the term "**exemplary**" or "**example**" means "serving as an example, instance, or illustration," and should not be construed as preferred or advantageous over other configurations disclosed herein.

As used herein, the terms **"about"** and "**approximately**", when used in conjunction with ranges of dimensions of particles, compositions of mixtures or other physical properties or characteristics, are meant to cover slight variations that may exist in the upper and lower limits of the ranges of dimensions so as to not exclude embodiments where on average most of the dimensions are satisfied but where statistically dimensions may exist outside this region. It is not the intention to exclude embodiments such as these from the present disclosure.

As used herein, the phrase **"non-Newtonian thickener"** or "non-Newtonian thickening agent" means a category of fluids whose viscosity (resistance to deformation or shear forces) is influenced by the shear stress and/or by the time this force is applied.

As used herein, the phrase "**critical strain force"** (also referred to as "**yield value")** refers to a term used in rheology in which the 'critical strain force' is the minimum force that must be applied to a material to induce viscous flow. The critical strain force is a measurable quantity (expressed in dynes/$cm^2$). More particularly, both phrases are referring to the same phenomenon which is that up to a certain force exerted (the critical strain force), a visco-elastic material will exhibit "solid like" or "elastic" properties, i.e. it will not have a net flow. Above the critical strain force (or above the "yield value") the same material will exhibit viscous properties, i.e. it will flow.

The present formulation strategy to find a replacement for surfactants and solvents was to identify some oil-absorbing and lipophilic ingredients (see below) with the potential to replace all or part of the surfactants and solvents usually involved in formulations of heavy duty hand cleansers.

Oil-absorbers such as polynorbornenes are used mainly in the rubber industry for anti-vibration (rail, building), anti-impact (personal protective equipment, shoe parts, bumpers) and grip improvement (toy tires, racing tires, transmission systems, transports systems for copiers, feeders, etc.). They are also useful for other applications such as oil-binding system with absorption capability (10 times of own weight) of hydrocarbons to treat pollutions from oil spills. It has been surprisingly found that the invention is able to provide high levels of skin cleansing efficacy with only 5% of norbornene polymer suspended in an aqueous gel.

The norbornene molecule carries a double bond which induces significant ring strain and significant reactivity: It is made by a Diels-Alder reaction of cyclopentadiene and ethylene:

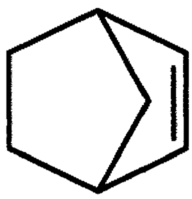 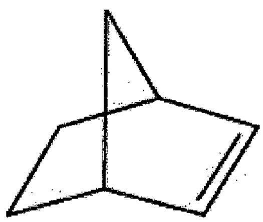

The IUPAC name of the molecule is bicyclo[2.2.1]hept-2-ene, its CAS Number is 498-66-8, and its chemical formula is $C_7H_{10}$. The norbornene molecule (also called norbornylene or norcamphene) is not toxic to humans and aquatic life. It is comprised of a cyclohexene ring bridged with a methylene group in the 'para' position.

In addition to norbornene, the present formulations instead of using norbornene includes derivatives of norbornene and may also include separately or in combination related bicyclics of norbornene including norbornadiene which has the same carbon skeleton but with two double bonds, and derivatives of norbornadiene and norbornane which is completely saturated without double bonds, and its derivatives.

Formulations may also contain, alone or in combination with norbornyl-related polymeric particles, other lipophilic absorbent particles such as cellulose acetate:

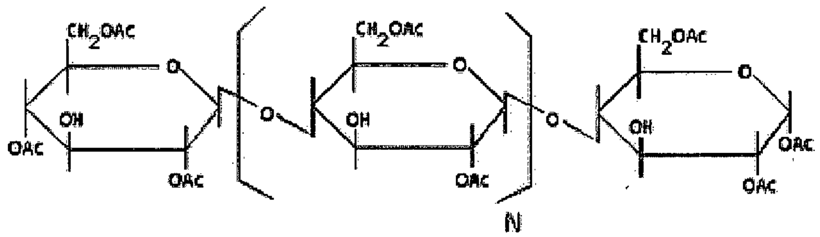

Cellulose acetate is also known as zyl, zylonite, Cellon and Rhodoid.

Formulations may also include aggregates of hydrophobic (fumed) silica which hydrophobic groups are alkyl or polydimethylsiloxane chains such as in the following structure:

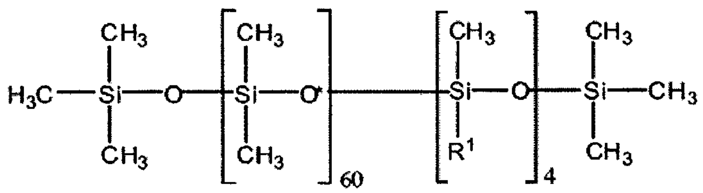

Fumed silica, also called 'pyrogenic silica' consists of amorphous three-dimensional particles, and is characterized by an extremely low density and a very high specific surface area (500 to 2000 $m^2/g$).

Norbornene based polymer particles have a size ranging from 50 to 1000 μm, which is an appropriate granulometry and the right hardness also serve as a scrubbing agent. Preferred norbornene particles are available from the manufacturers Astrotech Advanced Elastomerproducts GmbH. Such particles are microparticles e.g., the product Norsorex® APX.

An embodiment of the skin cleansing formulation comprises a gel and particles of a polymer comprised of any one of bicyclo[2.2.1]hept-2-ene, homopolymer (norbornene) and/or its derivatives. Other embodiments may contain norbornadiene and its derivatives or and norbornane and its derivatives.

Embodiments include formulations containing derivatives of norbornane, norbornene, norbornadiene include those in which a hydrogen atom of one more C-H bonds of norbornane, norbornene or norbornadiene is substituted with a hydrocarbyl group. The term "hydrocarbyl", when referring to groups attached to the remainder of a molecule, refers to a radical group made up of carbon and hydrogen atoms. Such groups include: C1-C20 hydrocarbyl groups that are straight chain and branched alkyl groups, C2-C20 alkenyl and alkynyl groups containing one or more or both type of said unsaturations, C3-C20 hydrocarbyl groups containing one or more rings, and which may also contain one or more alkenyl or alkynyl unsaturations. Examples of hydrocarbon groups include methyl, ethyl, n-propyl, i-propyl, n-butyl, i-butyl, t-butyl, n-pentyl, branched pentyl, hexyl, branched hexyl, heptyl, branched heptyl, octyl, branched octyl, and any of C9-, C10-, C11-, C12-, C13-, C14-, C15-, C16-, C17-, C18-, C19- and C20- straight chain and branched alkyl groups. Alkenyl and alkynyl groups include those corresponding to the foregoing alkyl groups of ethyl, n-propyl, i-propyl, etc. containing one or more double bonds (C=C) or triple bonds (C=C) or combinations of double and triple bonds. Cyclic hydrocarbyl groups include cyclopropyl, cycobutyl, cyclopentyl, norbornyl, and the like, and these may be appended by a diradical formed by replacing a hydrogen atom of a linear or branched hydrocarbyl group with such a cyclic group. Cyclic groups themselves can include one or more double and/or triple bonds.

In the context of this invention, the term "microparticle" refers to a solid or amorphous particle 1 μm to about 1 mm in diameter. Lipophilic particles of a formulation can extend to particles in the range from 1 μm to 3,000 μm, 10 μm to 3,000 μm, 10 μm to 2,000 μm, but more likely ranges are 10 μm to 1,000 μm, 20 μm to 900 μm or 40 to 900 μm or about 50 to about 800 μm. Particle size is readily determined by techniques well known in the art, such as e.g., photon correlation spectroscopy, laser diffractometry and/or scanning electron microscopy. The term "microparticles" means a collection of particles whose average size is less than 1 mm, more likely less than 800 μm. This includes collections having an average size in the range of from 50 μm to 800 μm, 50 μm to 700 μm, 100 μm to 700 μm, 150 μm to 700 μm, 200 μm to 700 μm, 250 μm to 700 μm, 300 μm to 700 μm, 400 μm to 700 μm, 50 μm to 600 μm, 100 μm to 600

μm, 150 μm to 600 μm, 200 μm to 600 μm, 250 μm to 600 μm, 300 μm to 600 μm, 350 μm to 600 μm, 100 μm to 500 μm, 200 μm to 500 μm, or 300 μm to 500, or collections having an average size of about 100 μm, 150 μm, 200 μm, 250 μm, 275 μm, 300 μm, 325 μm, 350 μm, 375 μm, 400 μm, 425 μm, 450 μm, 475 μm, 500 μm, 525 μm, 550 μm, 575 μm, 600 μm, 625 μm, 650 μm, 675 μm, or 700 μm.

"Lipophilic" microparticles or particles of compositions have an affinity for e.g., hydrocarbons and absorb these in use as part of the cleansing process. This has been found to reduce or even eliminate the need for a solvent, as the term is used in the art, as part of the cleanser. As mentioned above such cleaning solvents include white spirits, vegetable oils, esters, paraffin oil, and terpenes.

A particle is a solid particle that does not dissolve in a liquid such as water or toluene. Particles composed of monomeric units that are lipophilic when isolated are themselves said to be lipophilic particles. For the purposes of this definition, an isolated monomeric unit of e.g., the homopolymer polynorbornene can be obtained by replacing the single bonds to neighboring units of polymer by hydrogen atoms to obtain compound **A.**

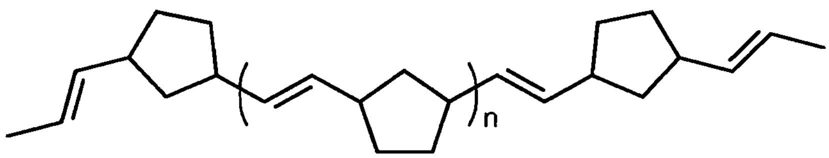

polynorbornene

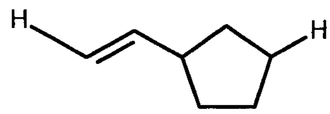

**A**

Compound **A** does not dissolve to an appreciable extent in or mix with water i.e., is not miscible with water, so is lipophilic, and so polymeric particles composed of the monomer are said to be lipophilic. Compound **A** does dissolve in or is miscible with a lipophilic solvent e.g., white spirits (petroleum distillates), paraffin oil, terpenes, etc. Fumed silica particles are often referred to in the art as hydrophobically-modified silica and are lipophilic particles. Norbornene has exemplary oil-binding capabilities with respect to hydrocarbons, absorbing up to ten times its own weight.

In another context, cleansing formulations are said to be substantially free of lipophilic solvents. Such solvents are not appreciably miscible with water i.e., less than 1 mg/ml (preferably less than 0.5 mg/ml) is miscible with water at 15°C, but such solvents are miscible with other lipophilic solvents such as white spirits, paraffin oil, terpenes, benzene, toluene, etc. A formulation is substantially free of lipophilic solvent when lipophilic solvent(s) make up no more than about 5 % w/w of the formulation, more preferably less than about 4 % w/w, 3 % w/w, 2 % w/w, 1 % w/w, 0.5% w/w, or less than 0.1 % w/w of a formulation. In the examples described here, no lipophilic solvent is added to the formulation, so these formulations contain at most trace amounts of such solvent(s) as may be present in components of the formulation as sourced. Such formulations are referred to as solvent-free formulations. As water is not a lipophilic liquid, but rather a hydrophilic liquid, it is not considered a solvent in this context.

The particles are suspended in the gel and present in a concentration from about 0.1 % to about 99 % w/w of the formulation. More preferably the polymer particles are present in a range from about 0.5 to about 20% w/w, and more preferably they are present in a range from about 1 to about 10% w/w. The particles have a size in a range from about 10 to about 3000 μm, and more preferably in the range from about 100 to 1000 μm.

The gel includes at least one non-Newtonian thickener able to provide the formulation with a yield value of 30 dynes/$cm^2$ or more, the non-Newtonian thickener being any one or combination of acrylates/C10-30 alkyl acrylate crosspolymer, carbomer, xanthan gum, guar gum, quaternised guar gum, alginate and bentonite, in a concentration of from about 0.01 to about 10% w/w of the formulation, more likely in a range from about 0.1 to about 10%, or 0.1 and 5%, or 0.1 and 4%, or 0.1 and 3%, or 0.1 and 2%, or 0.1 and 1% w/w of the gel formulation.

The non-Newtonian thickener able to provide the formulation with a yield value of 30 dynes/$cm^2$ or more, the non-Newtonian thickener being any one or combination of acrylates/C10-30 alkyl acrylate crosspolymer, carbomer, xanthan gum, guar gum, quaternised guar gum, alginate, bentonite.

In order to provide the structure to suspend the particles, the gel must also have a yield value superior or equal to 30 dynes / $cm^2$ associated or not to non-Newtonian rheological behaviour, and it must have more particularly plastic or thixotropic properties (pseudoplastic fluids being excluded from this requirement). The rheology of this form of gel must be viscoplastic (Casson, or Bingham, or dilatant, or thixotropic, or rheopectic fluids) with a yield value which is preferably greater than or equal to 30 dynes/$cm^2$. The Yield value is defined as that stress which must be applied before flow will start and, although related to viscosity, it its more dependent on the characteristics of the rheological additive used. Yield value has traditionally been determined by measuring the viscosity of the material at two speeds using a Brookfield Viscometer. As the speed of rotation of the spindle is increased materials that undergo shear thinning give a lower viscosity measurement.

The soap's yield value or the required yield value may be calculated as follows:

$$\text{Yield Value (in dynes/cm}^2) = (V_{0.5rpm} - V_{1rpm}) / 100$$

$$\text{Required Yield Value (in dynes/cm}^2) = [4/3\ R(D\text{-}Do)g]$$

Where:

*R = particle radius (cm)*
*D = particle density (g/cm$^3$) Do = medium density (glcm$^3$)*
*g = acceleration due to gravity = 980 cm/s$^2$*

The non-Newtonian thickener may be present in a range from about 0.1 to about 10% w/w, of the formulation. Viscosity can be in the range e.g., of from 500 to 100,000 cP (centipoise), more preferably in the range of 1,000 to 20,000, or 2,000 to 10,000 cP.

The formulation lipophilic particles may be present in a concentration from about 0.1 to about 95% w/w, or more preferably they may be present in a concentration from about 0.5 to about 20% w/w. More preferably the particles are present in a concentration from about 1 to about 10% w/w.

The formulation can additionally include scrubbing particles that are vegetable based, organic based and/or mineral based scrubbing agents. The vegetable based scrubbing particles may include any one or more of ground cornmeal, ground fruit stones, ground walnuts; the organic based scrubbing particles may include any one or more of wood dust, polyethylene and polypropylene; and the mineral based scrubbing particles may include one or both of pumice and ground shellfish.

The formulation may include at least one preservative having bacteriostatic and fongistatic properties. Non-limiting examples of the preservative includes any one or combination of benzoic acid, benzyl alcohol, bromo-nitro-propanediol, chloroxylenol, diazolidinyl urea, dehydroacetic acid, dichloro-benzyl alcohol, imadazolidinyl urea, iodopropynylbutyl carbamate, methyl-chloro-isothiazolinone and methyl-isothiazolinone, paraben methyl and/or ethyl and/or butyl and/or isobutyl, phenoxyethanol, poly(hexa-methylene biguanide)-hydrochloride, propionic acid, sodium hydroxymethyl glycinate and sorbic acid.

The formulation may include at least one chelating agent. Non-limiting examples of chelating agents include any one or combination of sodium tripolyphosphate, ethylenediaminetetraacetic acid (EDTA), diethylenetriaminepentaacetic acid (DTPA), N-(hydroxyethyl)-ethylenediaminetriacetic acid (HEDTA), 2-hydroxyethyliminodiacetic acid (HEIDA), aminobenzoic acid, iminodisuccinic acid, polyaspartic acid, gluconic acid, and salts thereof, in a concentration of from 0.01 to 5% w/w. More preferably the chelating agent is present from about 0.1 to about 0.5% w/w.

The formulation may include at least one skin conditioner. Non-limiting examples of the skin conditioner includes any one or combination of polyols (glycerin, polyglycerol, sorbitol, propylene glycol), allantoin, quaternised polymers or gums (polyquaterniums, dihydroxypropyl PEG-5 linoleammonium chloride, guar hydroxypropyltrimonium chloride, behentrimonium chloride), butylene glycol, hydrogenated vegetal oils, toxopherols, present in a concentration from about 0.01 to about 10% w/w. More preferably the at least one skin conditioner is present in a concentration from about 0.1 to about 5% w/w. Certain of these compounds e.g., polyquaternium-7 have surfactant properties, however, are included as part of a formulation for skin conditioning properties. As such, they are incorporated into a composition at a level at which they contribute little, if anything, to the cleansing properties of a formulation. In this sense, a formulation is said to be surfactant-free and to be substantially free of surfactant. Examples of such levels are less than 1 %, or less than 0.9 %, or less than 0.8 %, or less than 0.7 %, or less than 0.6 %, or less than 0.5 %, or less than 0.4 %, or less than 0.3 %, or less than 0.2 %, or less than 0.1 %, or less than 0.09 %, or less than 0.08 %, or less than 0.07 %, or less than 0.06 %, or less than 0.05 %, or less than 0.04 %, or less than 0.03 %, or less than 0.02 %, or about 1 %, or about 0.9 %, or about 0.8 %, or about 0.7 %, or about 0.6 %, or about 0.5 %, or about 0.4 %, or about 0.3 %, or about 0.2 %, or about 0.1 %, or about 0.09 %, or about 0.08 %, or about 0.07 %, or about 0.06 %, or about 0.05 %, or about 0.04 %, or about 0.03 %, or about 0.02 %, or about 0.01 % w/w of a formulation.

A formulation may include at least one hydrotrope, but is surfactant-free, or at least substantially free of surfactant as discussed above. Surfactants are compounds that lower surface tension between two liquids or a liquid and a solid and can act as detergents. One or more hydrotropes can be included in a cleansing a composition to aid stabilization of the gel. A hydrotrope, although amphiphilic like a surfactant, is distinct from a surfactant in this setting in that a hydrotrope does not self-aggregate as a surfactant. Non-limiting examples of the hydrotrope include any one or combination of sodium xylene sulfonate, ammonium xylene sulfonate, calcium xylene sulfonate, potassium xylene sulfonate, sodium toluene sulfonate, ammonium toluene sulfonate, sodium cumene sulfonate, ammonium cumene sulfonate, phosphate polyether ester, alkyldiphenyloxide disulfonate, present in a concentration of from about 0.01 to about 10% w/w.

More preferably the at least one hydrotrope is present in a concentration from about 0.1 to about 5% w/w.

The formulation may include at least one pH-adjuster for adjusting the pH of the formulation between about pH 4 and about pH 9, and more preferably between about pH 5 and pH 7. Non-limiting examples of the pH-adjuster include an acidic or alkaline pH-adjuster and is any one or combination of citric acid, phosphoric acid, lactic acid, tartaric acid, sodium hydroxide, potash, aminomethyl propanol, triethanolamine.

The formulation may include any one or combination of colouring dyes, pigments and perfumes.

The formulation may include water, or it may be waterless. A composition that is "substantially free of water" is one to which water has not been added during its formulation, so that it contains at most water contained in ingredients used to make up the composition. The term "waterless" means that a composition contains from 0 up to 5%, preferably less than 3%, most preferably less than 1 % by weight of water, based on the total weight of the composition.

## EXAMPLES

A simple preliminary test was conducted as follows: Three (3) test subjects washed their hands with soap and dried them well to remove any soiling already present on the skin. The testers soiled their hands with 0.4 g of a reference greasy stain called 'Black Gunge' having ingredients shown in **Table 1,** all around their hands, covering all areas up to their wrists.

**Table 1**

| Black Gunge Formulation | % w/w |
|---|---|
| Hydraulic Oil | 40 |
| Charcoal Powder | 20 |
| Hydrocarbon solvent (Premium Burning Oil®) | 10 |
| Sunflower seed oil | 5 |
| Kaolin | 5 |
| Lanolin Ester | 20 |

Without pre-wetting their hands, the test subjects applied 2 ml of a test-product containing ingredients shown in **Table 2** and rubbed it around hands for 30 seconds, insisting on the backs of the hands and between the fingers.

**Table 2**

| Ingredients | % w/w |
|---|---|
| Water | Qs 100 |
| Acrylates Copolymer (Aqua SF1®) | 6.00 |
| Norbonene polymer (Norsorex APX®) | 5.00 |
| Sodium hydroxide | Qs pH 7 +/- 0.5 |

They washed their hands were thoroughly to remove product and soiling and then dried.

The test was repeated with a very effective reference heavy duty hand cleanser 'Swarfega Orange®' from the Deb Group Ltd's international range. Eventual soiling left was assessed by a technician and results were scored with results shown in **Table 3.**

**Table 3**

| Sum of 1 and 2 | Final Score | Interpretation of Final Score |
|---|---|---|
| 0 | 5 | Complete removal of gunge |
| 40 | 4 | Some removal of gunge |
| 80 | 3 | Some removal of gunge |
| 120 | 2 | Some removal of gunge |
| 160 | 1 | Some removal of gunge |
| 200 | 0 | No removal of gunge |

Unexpectedly, an average final score of 4.66 was obtained for both test-product and reference product, which

means that the test-product was as effective as the reference heavy duty hand cleanser'Swarfega Orange®' which contains 15-20% of active matter (surfactant, polygrains and solvent).

**Table 4** shows non-limiting examples of water-based formulations.

**Table 4**

| Ingredients | Concentrations % w/w | | | | | |
|---|---|---|---|---|---|---|
| Water | Qs to 100 | | | | | |
| Norbornene polymer | 5 | 10 | 20 | 40 | 80 | 99 |
| Acrylic polymer | 0.40 | | | | | |
| Sodium cumene sulfonate | 1.00 | | | | | |
| Glycerin | 3.00 | | | | | |
| Polyquaternium-7 | 0.50 | | | | | |
| Phenoxyethanol | 1.00 | | | | | |
| Diazolidinyl urea | 0.50 | | | | | |
| Sodium imminodisuccinate | 0.40 | | | | | |
| sodium xylene sulfonate | 2.00 | | | | | |
| Perfume | 0.20 | | | | | |
| CI 19140 | 0.01 | | | | | |
| CI 42090 | 0.02 | | | | | |
| Sodium hydroxide or citric acid | Qs to pH 7+/-0.5 | | | | | |

**Tables 5** to **10** show non-limiting examples of water-less formulations. These tables 5 to 10 do not form part of the present invention.

**Table 5**

| Ingredients | Concentrations % w/w | | | | | |
|---|---|---|---|---|---|---|
| Norbornene polymer | 5 | 10 | 20 | 40 | 80 | 100 |
| Vegetable polygrains | Qs 100 | | | | | 0 |
| Sodium cumene sulfonate | 1.00 | | | | | 0 |
| Glycerin | 3.00 | | | | | 0 |
| Polyquaternium-7 | 0.50 | | | | | 0 |
| Perfume | 0.20 | | | | | 0 |
| sodium xylene sulfonate | 2.00 | | | | | 0 |

**Table 5**

| Ingredients | Concentrations % w/w | | | | | |
|---|---|---|---|---|---|---|
| Norbornene polymer | 5 | 10 | 20 | 40 | 80 | 100 |
| Vegetable polygrains | Qs 100 | Qs 100 | Qs 100 | Qs 100 | Qs 100 | 0 |
| Sodium cumene sulfonate | 1.00 | 1.00 | 1.00 | 1.00 | 1.00 | 0 |
| Glycerin | 3.00 | 3.00 | 3.00 | 3.00 | 3.00 | 0 |
| Polyquaternium-7 | 0.50 | 0.50 | 0.50 | 0.50 | 0.50 | 0 |
| Perfume | 0.20 | 0.20 | 0.20 | 0.20 | 0.20 | 0 |
| sodium xylene sulfonate | 2.00 | 2.00 | 2.00 | 2.00 | 2.00 | 0 |

**Table 6**

| Ingredients | Concentrations % w/w | | | | | |
|---|---|---|---|---|---|---|
| Cellulose acetate | 5 | 10 | 20 | 40 | 80 | 100 |
| Vegetable polygrains | Qs 100 | Qs 100 | Qs 100 | Qs 100 | Qs 100 | 0 |

(continued)

| Ingredients | Concentrations % w/w | | | | | |
|---|---|---|---|---|---|---|
| Sodium cumene sulfonate | 1.00 | 1.00 | 1.00 | 1.00 | 1.00 | 0 |
| Glycerin | 3.00 | 3.00 | 3.00 | 3.00 | 3.00 | 0 |
| Polyquaternium-7 | 0.50 | 0.50 | 0.50 | 0.50 | 0.50 | 0 |
| Perfume | 0.20 | 0.20 | 0.20 | 0.20 | 0.20 | 0 |
| Sodium xylene sulfonate | 2.00 | 2.00 | 2.00 | 2.00 | 2.00 | 0 |

**Table 7**

| Ingredients | Concentrations % w/w | | | | | |
|---|---|---|---|---|---|---|
| Hydrophobic fumed silica | 5 | 10 | 20 | 40 | 80 | 100 |
| Vegetable polygrains | Qs 100 | Qs 100 | Qs 100 | Qs 100 | Qs 100 | 0 |
| Sodium cumene sulfonate | 1.00 | 1.00 | 1.00 | 1.00 | 1.00 | 0 |
| Glycerin | 3.00 | 3.00 | 3.00 | 3.00 | 3.00 | 0 |
| Polyquaternium-7 | 0.50 | 0.50 | 0.50 | 0.50 | 0.50 | 0 |
| Perfume | 0.20 | 0.20 | 0.20 | 0.20 | 0.20 | 0 |
| Sodium xylene sulfonate | 2.00 | 2.00 | 2.00 | 2.00 | 2.00 | 0 |

**Table 8**

| Ingredients | Concentrations % w/w | | | | | |
|---|---|---|---|---|---|---|
| Norbornene polymer and cellulose acetate (50:50) | 5 | 10 | 20 | 40 | 80 | 100 |
| Vegetable polygrains | Qs 100 | Qs 100 | Qs 100 | Qs 100 | Qs 100 | 0 |
| Sodium cumene sulfonate | 1.00 | 1.00 | 1.00 | 1.00 | 1.00 | 0 |
| Glycerin | 3.00 | 3.00 | 3.00 | 3.00 | 3.00 | 0 |
| Polyquaternium-7 | 0.50 | 0.50 | 0.50 | 0.50 | 0.50 | 0 |
| Perfume | 0.20 | 0.20 | 0.20 | 0.20 | 0.20 | 0 |
| Sodium xylene sulfonate | 2.00 | 2.00 | 2.00 | 2.00 | 2.00 | 0 |

**Table 9**

| Ingredients | Concentrations % w/w | | | | | |
|---|---|---|---|---|---|---|
| Norbornene polymer and hydrophobic fumed silica (50:50) | 5 | 10 | 20 | 40 | 80 | 100 |
| Vegetable polygrains | Qs 100 | Qs 100 | Qs 100 | Qs 100 | Qs 100 | 0 |
| Sodium cumene sulfonate | 1.00 | 1.00 | 1.00 | 1.00 | 1.00 | 0 |
| Glycerin | 3.00 | 3.00 | 3.00 | 3.00 | 3.00 | 0 |
| Polyquaternium-7 | 0.50 | 0.50 | 0.50 | 0.50 | 0.50 | 0 |
| Perfume | 0.20 | 0.20 | 0.20 | 0.20 | 0.20 | 0 |
| Sodium xylene sulfonate | 2.00 | 2.00 | 2.00 | 2.00 | 2.00 | 0 |

**Table 10**

| Ingredients | Concentrations % w/w | | | | | |
|---|---|---|---|---|---|---|
| Norbornene polymer and cellulose acetate and hydrophobic fumed silica (1/3:1.3:1/3) | 5 | 10 | 20 | 40 | 80 | 100 |
| Vegetable polygrains | Qs 100 | Qs 100 | Qs 100 | Qs 100 | Qs 100 | 0 |
| Sodium cumene sulfonate | 1.00 | 1.00 | 1.00 | 1.00 | 1.00 | 0 |
| Glycerin | 3.00 | 3.00 | 3.00 | 3.00 | 3.00 | 0 |

(continued)

| Ingredients | Concentrations % w/w | | | | | |
|---|---|---|---|---|---|---|
| Polyquaternium-7 | 0.50 | 0.50 | 0.50 | 0.50 | 0.50 | 0 |
| Perfume | 0.20 | 0.20 | 0.20 | 0.20 | 0.20 | 0 |
| Sodium xylene sulfonate | 2.00 | 2.00 | 2.00 | 2.00 | 2.00 | 0 |

**Claims**

**1.** Use of a formulation as a hand cleanser, the formulation comprising:

a gel; and
lipophilic polymeric particles including a polymer of a norbornane, norbornene, norbornadiene, or a derivative of norbornane, norbornene or norbornadiene, in which a hydrogen atom of one or more C-H bonds of the norbornane, norbornene or norbornadiene is substituted with a hydrocarbyl group; or

cellulose acetate; or
aggregates of fumed silica; or a combination thereof;
the particles being suspended in the gel and present in a concentration from about 0.1 % w/w to about 99% w/w of the formulation, wherein:

the gel comprises at least one non-Newtonian thickener present in an amount sufficient to provide the formulation with a yield value of at least 30 dynes/$cm^2$ (3 Pa);
the formulation is optionally free of lipophilic solvent; and
the formulation is optionally free of surfactant.

**2.** Use according to claim 1, wherein the at least one non-Newtonian thickener is any one or combination of acrylates/C10-30 alkyl acrylate crosspolymer, carbomer, xanthan gum, guar gum, quaternised guar gum, alginate, bentonite, in a concentration of from about 0.01 to about 10% w/w of the formulation, or in a range from about 0.1 to about 10% w/w of the formulation, and/or wherein the polymer particles are present in the formulation in a range from about 0.5 o about 20% w/w of the formulation.

**3.** Use according to any one of claims 1 to 2, wherein the formulation further comprises:

a. scrubbing particles in a concentration from about 0.1 to about 95% w/w of the formulation, and/or wherein said scrubbing particles are any one or any combination of vegetable based, organic based and mineral based scrubbing agents, and/or,
b. at least one preservative having bacteriostatic and fongistatic properties, and/or
c. at least one chelating agent, in a concentration of from 0.01 to 5% w/w of the formulation, and/or
d. at least one skin conditioner, and/or
e. at least one hydrotope, and/or
f. any one or a combination of colouring dyes, pigments and perfumes.

**4.** Use according to any one of claims 1 to 3, wherein said lipophilic particles have a size in a range from about 10 to about 3000 μm, and/or wherein said lipophilic particles have an average size in the range of from 50 μm to 2000 μm.

**5.** Use according to any one of claims 1 to 4, wherein said formulation is substantially free of lipophilic solvent, and/or surfactant, and/or water.

**6.** Use according to any one of claims 1 to 5, wherein the formulation further comprises water,
and preferably further comprises at least one pH-adjuster and wherein the formulation has a pH between about 4 and about 9, and even more preferably wherein the pH-adjuster is an acidic or alkaline pHadjuster and is any one or a combination of citric acid, phosphoric acid, lactic acid, tartaric acid, sodium hydroxide, potash, aminomethyl propanol, and triethanolamine.

**7.** A skin cleansing formulation, comprising a gel; and lipophilic particles comprising cellulose acetate; or lipophilic

particles comprising aggregates of fumed silica, the particles being suspended in the gel and present in a concentration from about 0.1% w/w to about 99% w/w of the formulation, wherein the gel comprises at least one non-Newtonian thickener present in an amount sufficient to provide the formulation with a yield value of at least 30 dynes/cm$^2$ (3 Pa); the formulation is optionally free of lipophilic solvent, and optionally free of surfactant.

**8.** The formulation according to claim 7, wherein the at least one non-Newtonian thickener is any one or combination of acrylates/C10-30 alkyl acrylate crosspolymer, carbomer, xanthan gum, guar gum, quaternised guar gum, alginate, bentonite, in a concentration of from about 0.01 to about 10% w/w of the formulation, and/or wherein the non-Newtonian thickener is present in a range from about 0.1 to about 5% w/w of the formulation, and/or wherein the polymer particles are present in the formulation in a range from about 0.5 to about 20% w/w of the formulation.

**9.** The formulation according to claim 7 or 8, further comprising:

a. scrubbing particles in a concentration from about 0.1 to about 95% w/w of the formulation, and/or wherein said scrubbing particles are any one or any combination of vegetable based, organic based and mineral based scrubbing agents, and/or
b. at least one preservative having bacteriostatic and fongistatic properties, and/or
c. at least one chelating agent, and/or
d. at least one skin conditioner, and/or,
e. further comprising at least one hydrotrope, and/or
f. any one or a combination of colouring dyes, pigments and perfumes.

**10.** The formulation according to any one of claims 7 to 9, wherein said lipophilic particles have a size in a range from about 10 to about 3000 μm, and/or wherein said lipophilic particles have an average size in the range of from 50 μm to 2000 μm.

**11.** The formulation according to any one of claims 7 to 10, wherein the formulation includes water, and further comprising at least one pH-adjuster and wherein the formulation has a pH between about 4 and about 9, and/or wherein the pH-adjuster is an acidic or alkaline pH adjuster and is any one or a combination of citric acid, phosphoric acid, lactic acid, tartaric acid, sodium hydroxide, potash, aminomethyl propanol, and triethanolamine.

**12.** The formulation according to any one of claims 7 to 11, wherein the lipophilic particles comprise: a polymer of a norbornane, norbornene, norbornadiene, or a derivative of norbornane, norbornene or norbornadiene; cellulose acetate; aggregates of fumed silica, or any combination thereof.

**13.** The formulation as defined by any one of claims 7 to 12, wherein said formulation is for use as a hand cleanser.

## Patentansprüche

**1.** Verwendung einer Formulierung als Handreiniger, wobei die Formulierung folgendes umfasst:

Ein Gel; und
lipophile Polymerpartikel mit einem Norbornan-, Norbornen-, Norbornadien-Polymer oder einem Norbornan-, Norbornen- oder Norbornadien-Derivat, bei dem ein Wasserstoffatom aus einer oder mehreren C-H-Bindungen des Norbornan, Norbornen oder Norbornadien mit einer Hydrocarbylgruppe substituiert ist; oder

Celluloseacetat; oder
Aggregate von pyrogener Kieselsäure; oder eine Kombination daraus;
wobei die Partikel im Gel suspendiert sind und in einer Konzentration zwischen ca. 0,1 und ca. 99 Gew.-% der Formulierung vorliegen, wobei:

das Gel mindestens ein nicht-Newtonsches Verdickungsmittel umfasst, das in einer Menge vorliegt, die ausreicht, um der Formulierung einen Fließwert von mindestens 30 dyne/cm$^2$ (3 Pa) zu verleihen;
wobei die Formulierung optional frei von lipophilem Lösungsmittel ist; und
die Formulierung optional frei von Tensiden ist.

**2.** Verwendung gemäß Anspruch 1, bei der das mindestens eine nicht-Newtonsche Verdickungsmittel eines von oder

eine Kombination von Acrylaten/C10-30 Alkylacrylat-Crosspolymer, Carbomer, Xanthangummi, Guarkernmehl, quaternisiertes Guarkernmehl, Alginat, Bentonit, in einer Konzentration zwischen ca. 0,01 bis ca. 10 Gew.-% der Formulierung, oder in einem Bereich zwischen ca. 0,1 und ca, 10 Gew.-% der Formulierung, ist, und/oder bei der die Polymerpartikel in der Formulierung in einem Bereich zwischen ca. 0,5 bis ca. 20 Gew.-% der Formulierung vorliegen.

**3.** Verwendung gemäß einem der Ansprüche 1 bis 2, bei der die Formulierung des Weiteren folgendes umfasst:

a. Scheuerpartikel in einer Konzentration von ca. 0,1 bis ca. 95 Gew.-% der Formulierung, und/oder bei der die Scheuerpartikel eine beliebige oder jedwede Kombination aus pflanzlichen, organischen oder mineralischen Scheuerpartikeln ist, und/oder
b. mindestens einen Konservierungsstoff mit bakterienhemmenden und fungistatischen Eigenschaften; und/oder
c. mindestens einen Chelatbildner, in einer Konzentration zwischen 0,01 bis 5 Gew.-% der Formulierung, und/oder
d. mindestens ein Hautpflegemittel, und/oder
e. mindestens ein Hydrotop, und/oder
f. einen beliebigen oder eine Kombination aus Farbstoffen, Pigmenten und Duftstoffen.

**4.** Verwendung gemäß einem der Ansprüche 1 bis 3, bei der die lipophilen Teilchen eine Größe im Bereich zwischen ca. 10 bis ca. 3000 μm haben, und/oder bei der die lipophilen Teilchen eine Durchschnittsgröße im Bereich zwischen ca. 50 μm und 2000 μm haben.

**5.** Verwendung gemäß einem der Ansprüche 1 bis 4, bei der die Formulierung im Wesentlichen frei von lipophilem Lösungsmittel und/oder Tensid und/oder Wasser ist.

**6.** Verwendung gemäß einem der Ansprüche 1 bis 5, bei der die Formulierung des Weiteren Wasser umfasst, und vorzugsweise des Weiteren mindestens ein Mittel zur pH-Einstellung umfasst, und bei der die Formulierung einen pH-Wert zwischen ca. 4 und ca. 9 hat, und bei der noch bevorzugter das Mittel zur pH-Einstellung ein saures oder basisches Mittel zur pH-Einstellung ist und eine beliebige oder eine Kombination aus Zitronensäure, Phosphorsäure, Milchsäure, Weinsäure, Natriumhydroxid, Kaliumkarbonat, Aminomethylpropanol und Triethanolamin ist.

**7.** Hautreinigerformulierung, umfassend ein Gel; und lipophile Polymerpartikel umfassend Celluloseacetat; oder lipophile Partikel umfassend Aggregate von pyrogener Kieselsäure; wobei die Partikel im Gel suspendiert sind und in einer Konzentration von zwischen ca. 0,1 und ca. 99 Gew.-% der Formulierung vorliegen, wobei das Gel mindestens ein nicht-Newtonsches Verdickungsmittel umfasst, das in einer Menge vorliegt, die ausreicht, um der Formulierung einen Fließwert von mindestens 30 dyne/$cm^2$ (3 Pa) zu verleihen; die Formulierung optional frei von lipophilem Lösungsmittel sowie optional frei von Tensiden ist.

**8.** Formulierung gemäß Anspruch 7, bei der das mindestens eine nicht-Newtonsche Verdickungsmittel eines von oder eine Kombination von Acrylaten/C10-30 Alkylacrylat-Crosspolymer, Carbomer, Xanthangummi, Guarkernmehl, quaternisiertes Guarkernmehl, Alginat, Bentonit, in einer Konzentration zwischen ca. 0,01 bis ca. 10 Gew.-% der Formulierung, ist, und/oder bei der das nicht-Newtonsche Verdickungsmittel in einem Bereich von ca. 0,1 bis ca. 5 Gew.-% der Formulierung vorliegt, und/oder bei der die Polymerpartikel in der Formulierung in einem Bereich zwischen ca. 0,5 und ca. 20 Gew.-% der Formulierung vorliegen.

**9.** Formulierung gemäß Anspruch 7 oder 8, des Weiteren umfassend:

a. Scheuerpartikel in einer Konzentration von ca. 0,1 bis ca. 95 Gew.-% der Formulierung, und/oder bei der die Scheuerpartikel ein beliebiges oder jedwede Kombination aus pflanzlichen, organischen oder mineralischen Scheuerpartikeln sind, und/oder
b. mindestens einen Konservierungsstoff mit bakterienhemmenden und fungistatischen Eigenschaften; und/oder
c. mindestens einen Chelatbildner, und/oder
d. mindestens ein Hautpflegemittel, und/oder
e. des Weiteren umfassend mindestens ein Hydrotop, und/oder
f. ein beliebiges oder eine Kombination aus Farbstoffen, Pigmenten und Duftstoffen.

**10.** Formulierung gemäß einem der Ansprüche 7 bis 9, bei der die lipophilen Teilchen eine Größe im Bereich zwischen

ca. 10 bis ca. 3000 μm haben, und/oder bei der die lipophilen Teilchen eine Durchschnittsgröße im Bereich zwischen ca. 50 μm und 2000 μm haben.

**11.** Formulierung gemäß einem der Ansprüche 7 bis 10, bei der die Formulierung Wasser umfasst, und des Weiteren mindestens ein Mittel zur pH-Einstellung umfasst, und bei der die Formulierung einen pH-Wert zwischen ca. 4 und ca. 9 hat, und/oder bei der das Mittel zur pH-Einstellung ein saures oder basisches Mittel zur pH-Einstellung ist und eine beliebige oder eine Kombination aus Zitronensäure, Phosphorsäure, Milchsäure, Weinsäure, Natriumhydroxid, Kaliumkarbonat, Aminomethylpropanol und Triethanolamin ist.

**12.** Formulierung gemäß einem der Ansprüche 7 bis 11, bei der die lipophilen Partikel folgendes umfassen:

Ein Norbornan-, Norbornen-, Norbornadien-Polymer oder ein Norbornan-, Norbornen- oder Norbornadien-Derivat, Celluloseacetat; Aggregate von pyrogener Kieselsäure, oder jedwede Kombinationen daraus.

**13.** Formulierung gemäß einem der Ansprüche 7 bis 12, bei der die Formulierung zur Verwendung als Handreiniger dient.

**Revendications**

**1.** Utilisation d'une formulation en tant que nettoyant pour les mains, la formulation comprenant :

un gel ; et
des particules polymériques lipophiles incluant un polymère d'un norbornane, d'un norbornène, d'un norbornadiène, ou un dérivé de norbornane, de norbornène ou de norbornadiène, dans lesquelles un atome d'hydrogène d'une ou plusieurs liaisons C-H du norbornane, du norbornène ou du norbornadiène est substitué par un groupe hydrocarbyle ; ou

de l'acétate de cellulose ; ou
des agrégats de silice sublimée ; ou une combinaison de ceux-ci ;
les particules étant en suspension dans le gel et présentes en une concentration d'environ 0,1 % en p/p à environ 99 % en p/p de la formulation, dans laquelle :

le gel comprend au moins un épaississant non newtonien présent en une quantité suffisante pour conférer à la formulation un seuil d'écoulement d'au moins 30 dynes/$cm^2$ (3 Pa) ;
la formulation est facultativement exempte de solvant lipophile ; et
la formulation est facultativement exempte de tensioactif.

**2.** Utilisation selon la revendication 1, dans laquelle l'au moins un épaississant non newtonien est l'un quelconque ou une combinaison d'acrylates/polymère d'acrylate d'alkyle réticulé en C10 à C30, de carbomère, de gomme xanthane, de gomme de guar, de gomme de guar quaternisée, d'alginate, de bentonite, en une concentration d'environ 0,01 à environ 10 % en p/p de la formulation, ou dans une plage d'environ 0,1 à environ 10 % en p/p de la formulation, et/ou dans laquelle les particules de polymère sont présentes dans la formulation suivant une plage d'environ 0,5 à environ 20 % en p/p de la formulation.

**3.** Utilisation selon l'une quelconque des revendications 1 à 2, dans laquelle la formulation comprend en outre :

a. des particules purifiantes en une concentration d'environ 0,1 à environ 95 % en p/p de la formulation, et/ou dans laquelle lesdites particules purifiantes sont l'un quelconque ou une combinaison quelconque d'agents purifiants à base végétale, à base organique ou à base minérale, et/ou,
b. au moins un conservateur ayant des propriétés bactériostatiques et fongistatiques, et/ou
c. au moins un agent chélatant, en une concentration de 0,01 à 5 % en p/p de la formulation, et/ou
d. au moins un revitalisant pour la peau, et/ou
e. au moins un hydrotrope, et/ou
f. l'un quelconque ou une combinaison de colorants, pigments et parfums.

**4.** Utilisation selon l'une quelconque des revendications 1 à 3, dans laquelle lesdites particules lipophiles ont une taille dans une plage d'environ 10 à environ 3 000 μm, et/ou dans laquelle lesdites particules lipophiles ont une taille moyenne dans la plage de 50 μm à 2 000 μm.

**5.** Utilisation selon l'une quelconque des revendications 1 à 4, dans laquelle ladite formulation est sensiblement exempte de solvant lipophile, et/ou de tensioactif et/ou d'eau.

**6.** Utilisation selon l'une quelconque des revendications 1 à 5, dans laquelle la formulation comprend en outre de l'eau, et de préférence comprend en outre au moins un agent d'ajustement du pH et dans laquelle la formulation a un pH situé entre environ 4 et environ 9, et encore plus préférablement dans laquelle l'agent d'ajustement du pH est un agent d'ajustement du pH acide ou alcalin et est l'un quelconque ou une combinaison d'acide citrique, d'acide phosphorique, d'acide lactique, d'acide tartrique, d'hydroxyde de sodium, de potasse, de propanol aminométhylique et de triéthanolamine.

**7.** Formulation nettoyante pour la peau, comprenant un gel ; et des particules lipophiles comprenant de l'acétate de cellulose ; ou des particules lipophiles comprenant des agrégats de silice sublimée, les particules étant en suspension dans le gel et étant présentes en une concentration d'environ 0,1 % en p/p à environ 99 % en p/p de la formulation, dans laquelle le gel comprend au moins un épaississant non newtonien présent en une quantité suffisante pour conférer à la formulation un seuil d'écoulement d'au moins 30 dynes/$cm^2$ (3 Pa) ; la formulation est facultativement exempte de solvant lipophile, et facultativement exempte de tensioactif.

**8.** Formulation selon la revendication 7, dans laquelle l'au moins un épaississant non newtonien est l'un quelconque ou une combinaison d'acrylates/polymère d'acrylate d'alkyle réticulé en C10 à C30, de carbomère, de gomme xanthane, de gomme de guar, de gomme de guar quaternisée, d'alginate, de bentonite, en une concentration d'environ 0,01 à environ 10 % en p/p de la formulation, et/ou dans laquelle l'épaississant non newtonien est présent dans une plage d'environ 0,1 à environ 5 % en p/p de la formulation, et/ou dans laquelle les particules de polymère sont présentes dans la formulation suivant une plage d'environ 0,5 à environ 20 % en p/p de la formulation.

**9.** Formulation selon la revendication 7 ou 8, comprenant en outre :

a. des particules purifiantes en une concentration d'environ 0,1 à environ 95 % en p/p de la formulation, et/ou dans laquelle lesdites particules purifiantes sont l'un quelconque ou une combinaison quelconque d'agents purifiants à base végétale, à base organique ou à base minérale, et/ou
b. au moins un conservateur ayant des propriétés bactériostatiques et fongistatiques, et/ou
c. au moins un agent chélatant, et/ou
d. au moins un revitalisant pour la peau, et/ou,
e. comprenant en outre au moins un hydrotrope, et/ou
f. l'un quelconque ou une combinaison de colorants, pigments et parfums.

**10.** Formulation selon l'une quelconque des revendications 7 à 9, dans laquelle lesdites particules lipophiles ont une taille dans une plage d'environ 10 à environ 3 000 μm, et/ou dans laquelle lesdites particules lipophiles ont une taille moyenne dans la plage de 50 μm à 2 000 μm.

**11.** Formulation selon l'une quelconque des revendications 7 à 10, dans laquelle la formulation inclut de l'eau, et comprenant en outre au moins un agent d'ajustement du pH et dans laquelle la formulation a un pH situé entre environ 4 et environ 9, et/ou dans laquelle l'agent d'ajustement du pH est un agent d'ajustement du pH acide ou alcalin et est l'un quelconque ou une combinaison d'acide citrique, d'acide phosphorique, d'acide lactique, d'acide tartrique, d'hydroxyde de sodium, de potasse, de propanol aminométhylique et de triéthanolamine.

**12.** Formulation selon l'une quelconque des revendications 7 à 11, dans laquelle les particules lipophiles comprennent : un polymère d'un norbornane, d'un norbornène, d'un norbornadiène, ou un dérivé de norbornane, de norbornène ou de norbornadiène ; de l'acétate de cellulose ;
des agrégats de silice sublimée, ou toute combinaison de ceux-ci.

**13.** Formulation telle que définie par l'une quelconque des revendications 7 à 12, dans laquelle ladite formulation est destinée à être utilisée en tant que nettoyant pour les mains.